# EUROPÄISCHE PATENTSCHRIFT

(11) **EP 0 417 597 B1**
(45) Veröffentlichungstag und Bekanntmachung des Hinweises auf die Patenterteilung: **05.04.1995**
(21) Anmeldenummer: 90116918.5
(22) Anmeldetag: 04.09.1990
(51) Int. Cl.: C07C 67/38, C07C 69/66

(54) **Verfahren zur Herstellung von 2-Formylbuttersäuremethylester**
Method for the production of 2-formylbutyric acid methyl ester
Procédé pour la fabrication de l'ester méthylique de l'acide formyl-2-butyrique

(30) Priorität: 15.09.1989 DE 3930886
(43) Veröffentlichungstag der Anmeldung: 20.03.1991
(73) Patentinhaber: HOECHST AKTIENGESELLSCHAFT, 65926 Frankfurt am Main (DE)
(72) Erfinder: Weber, Jürgen, Dr. Dipl.-Chem., D-4200 Oberhausen 11 (DE); Lappe, Peter, Dr. Dipl.-Chem., D-4220 Dinslaken (DE); Springer, Helmut, Dipl.-Ing., D-4200 Oberhausen 11 (DE)

(56) Entgegenhaltungen:
- EP-A- 0 306 094
- BULLETIN OF THE CHEMICAL SOCIETY OF JAPAN. vol. 54, 1981, TOKYO JP Seiten 3799- 3805; T. Okano et al.: "Application of the water gas shift reaction I.Hydrogenation and hydroformylation reactions of olefins with carbon monoxide...
- BULLETIN OF THE CHEMICAL SOCIETY OF JAPAN. vol. 50, 1977, TOKYO JP Seiten 2351- 2357; M. Tanaka et al.: "Diphosphine-rhodium complex catalysed hydroformulation of alpha, beta-unsaturated esters"
- BRENNSTOFF CHEMIE. vol. 48, no. 2, 1967, ESSEN DE Seiten 46 - 52; J. Falbe et al.: "Hydroformylierung ungesättigter Carbonsäureester mit Rhodium-Katalysatoren"

## Beschreibung

Die Erfindung betrifft ein Verfahren zur Herstellung von 2-Formylbuttersäuremethylester durch Umsetzung von Crotonsäuremethylester mit Wasserstoff und Kohlenmonoxid.

2-Formylbuttersäureester sind wertvolle Zwischenprodukte in chemischen Synthesen. Durch Reduktion können sie in Hydroxyverbindungen überführt werden, die z.B. zur Herstellung von Polyestern verwendet werden. Die Umsetzung mit Ammoniak und Wasserstoff ergibt Ester von Aminomethylbuttersäuren und die Oxidation der Formylbuttersäureester führt zu Halbestern von Dicarbonsäuren.

Die Hydroformylierung von Crotonsäureestern führt stets zu Produktgemischen, die im wesentlichen 2-, 3-, und 4-Formylbuttersäureester und durch Hydrierung des Crotonsäureesters auch Buttersäureester enthalten. Die Umsetzung ist wiederholt untersucht worden.

So berichten Adkins et al. in J.Am.Chem.Soc. 71 (1949), S. 3051 ff über die Reaktion des Crotonsäureethylesters mit Wassergas (CO : H₂ = 1 : 1) bei 120 bis 125°C und einem Gesamtdruck von 200 bis 300 atm in Gegenwart von Kobalt als Katalysator und Benzol als Reaktionsmedium. Die Umsetzung ergibt in einer Ausbeute von 71 % 3-Formylbuttersäureethylester.

Nach Piacenti et al., Ullmanns Encyklopädie der technischen Chemie 62, Bd. 13, S. 65, erhält man, offensichtlich in Gegenwart eines Co-Katalysators, aus Ethylcrotonat in 70 %iger Ausbeute 2 -, 3- und 4-Formylbuttersäureethylester im Mengenverhältnis von 15 : 15 : 70.

Falbe et al. haben in Brennstoff Chemie 48, (1967), S. 46 ff die Ergebnisse der Hydroformylierung von Crotonsäureethylester in Gegenwart von 1 Gew.-% Rh₂O₃ in einer zweistufigen Reaktion publiziert. So resultiert bei 135°C und 200 atm Druck in der ersten Stufe und 200°C und 300 atm Druck in der zweiten Stufe ein Reaktionsgemisch, das im wesentlichen aus β-Methyl-γ-butyrolacton, Buttersäureethylester, δ-Valerolacton und α-Hydroxymethylbuttersaureethylester besteht.

Lai und Ucciani (Adv.Chem.Ser. 1974, (132), S.1 ff) haben Methylcrotonat unter verschiedenen Bedingungen hydroformyliert und festgestellt, daß die Selektivität der Reaktion bei Verwendung von Rhodium als Katalysator gegenüber der durch Kobalt katalysierten Umsetzung abnimmt. Die Selektivität wird noch geringer, wenn Rhodium zusammen mit Triphenylphosphin angewandt wird. Hauptprodukt bei der durch Kobalt katalysierten Umsetzung ist 4-Formylbuttersäuremethylester. Rhodium allein ergibt als Hauptprodukt die 3-Formylverbindung und Rhodium mit Triphenylphosphin liefert vorwiegend die 2-Formylverbindung, jedoch ist die Selektivität der Reaktion bei Verwendung von Rhodiumkatalysatoren völlig unzureichend.

Nach Tanaka et al., Bull.Chem.Soc.Jap. 50 (1977), 2351 ff hängt die Produktverteilung bei der Hydroformylierung von Methylcrotonat in Gegenwart von Rhodium sehr stark von der Art der verwendeten Liganden ab. Bei Einsatz von Triphenylphosphin als Ligand wird als Hauptprodukt in mäßiger Ausbeute 3-Formylbuttersäureester gebildet, die 2-Formylverbindung entsteht lediglich in untergeordneter Menge. Mit (H₅C₆)₂P(CH₂)₄P(C₆H₅)₂ als Ligand erhält man bevorzugt 2-Formylbuttersäureethylester.

Schließlich haben Okano et al., Bull.Chem.Soc.Jap. 54 (1981), 3799 ff gefunden, daß bei der Umsetzung von Crotonsäuremethylester mit Wasser in Gegenwart von Rhodium-Phosphin-Komplex-Verbindungen als Katalysatoren auf Grund des sich einstellenden Wassergas-Gleichgewichts in der Hauptsache Buttersäure und daneben in kleinen Mengen 2- und 3-Formylbuttersäuremethylester gebildet werden. Auch dieser Reaktionsweg eröffnet nicht die Möglichkeit zu einer wirtschaftlich befriedigenden Herstellung von 2-Formylbuttersäuremethylester in technischem Maßstab.

Es stellt sich daher die Aufgabe, ein Verfahren zu entwickeln, das die Hydroformylierung von Crotonsäuremethylester zu 2-Formylbuttersäuremethylester mit hoher Ausbeute und hoher Selektivität und unter Verwendung leicht zugänglicher Katalysatoren erlaubt.

Diese Aufgabe wird gelöst durch ein Verfahren zur Herstellung von 2-Formylbuttersäuremethylester durch Hydroformylierung von Crotonsäuremethylester in Gegenwart Rhodium und Tri-n-butylphosphin oder Triphenylphosphin enthaltender Katalysatoren. Es ist dadurch gekennzeichnet, daß die Umsetzung bei Temperaturen von 80 bis 120°C, Drücken von 20 bis 30 MPa, einer Rhodium-Konzentration von 10 bis 500 ppm, bezogen auf eingesetzten Crotonsäuremethylester, und in einem organischen Lösungsmittel als Reaktionsmedium erfolgt.

Als Ausgangsverbindung zur Herstellung von 2-Formylbuttersäuremethylester setzt man Crotonsäuremethylester ein. Er kann in reiner Form, d.h. destilliert angewendet werden. Es hat sich jedoch gezeigt, daß ohne Nachteil für Ausbeute und Selektivität auch Rohprodukte eingesetzt werden können, die neben Crotonsäuremethylester noch andere Bestandteile enthalten. So geht man zum Beispiel mit Erfolg unmittelbar von Gemischen aus, die bei der Veresterung von Crotonsäure mit Methylalkohol anfallen und 40 bis 60 Gew.-% Crotonsäuremethylester, 30 bis 40 Gew.-% Methanol und 5 bis 15 Gew.-% Wasser enthalten.

Kohlenmonoxid und Wasserstoff setzt man im allgemeinen in Form von Synthesegas ein, das durch partielle Oxidation von kohlenstoffhaltigem Material in Gegenwart von Wasser erhalten wird. Es enthält Kohlenmonoxid und Wasserstoff etwa im Verhältnis 1:1. Aber auch Gemische, in denen eine der Komponenten im Überschuß vorhanden ist, können mit Erfolg als Reaktionspartner verwendet werden. Bewährt haben sich in der Praxis CO/H₂-Gemische, die 0,8 bis 1,2 mol CO je mol H₂ enthalten. Es versteht sich von selbst, daß Verunreinigungen, die zu einer Vergiftung des Katalysators führen können, aus dem Gas entfernt werden müssen. So kann zum Beispiel Schwefel nur bis zu einer maximalen Konzentration von etwa 2 ppm toleriert werden.

Die Umsetzung der Ausgangsstoffe erfolgt in Gegenwart eines aus Rhodium und Tri-n-butylphosphin oder Triphenylphosphin bestehenden Katalysatorsystems. Die Konzentration des Rhodiums, bezogen auf den ursprünglich eingesetzten Crotonsäuremethylester, beträgt 10 bis 500 ppm, vorzugsweise 50 bis 200 ppm und insbesondere 80 bis 120 ppm. Das Rhodium wird als Metall, zweckmäßig in fein verteilter Form, oder als Verbindung eingesetzt. In der Praxis bewahrt hat sich als Ausgangssubstanz für den Katalysator insbesondere Rh-2-ethylhexanoat. Der zweite Bestandteil des Katalysatorsystems ist ein organisches Phosphin. Hierunter sind Alkyl- und Arylphosphine zu verstehen. Besonders bewährt haben sich Tri-n-butylphosphin und Triphenylphosphin. Je g-At Rhodium wendet man 1 bis 50, insbesondere 2 bis 20 mol Phosphin an. Das Katalysatorsystem kann dem Reaktionsgemisch formiert zugesetzt werden. In diesem Fall gewinnt man es in einem eigenen Verfahrensschritt, unabhängig von der eigentlichen Reaktion. Mit gleich gutem Ergebnis läßt sich der Katalysator aber auch in situ herstellen, also in dem Reaktionsgemisch und unter Hydroformylierungsbedingungen.

Ein wesentliches Merkmal der Erfindung ist die Einhaltung bestimmter Druck- und Temperaturbereiche während der Hydroformylierung, nämlich 80 bis 120°C und 20 bis 30 MPa. Zweckmäßig ist es, Temperaturen von 90 bis 110°C und insbesondere von 90 bis 100°C und Drücke von 200 bis 300 bar anzuwenden. Höhere Temperaturen als die vorstehend genannten führen zu verstärkter Hydrierung, niedrigere Temperaturen zu einer Minderung des Umsatzes.

Schließlich ist der neue Prozeß durch die Anwendung von organischen Lösungsmitteln als Reaktionsmedium charakterisiert. Besonders bewahrt haben sich aliphatische Kohlenwasserstoffe, vorzugsweise wird Cyclohexan eingesetzt. Der Anteil des Reaktionsmediums am Reaktionsgemisch kann in weiten Bereichen variieren und von 20 bis 80 Gew.-% betragen. Bevorzugt werden etwa 50 Gew.-%.

Das erfindungsgemäße Verfahren kann diskontinuierlich oder kontinuierlich durchgeführt werden. Bei diskontinuierlicher Arbeitsweise gibt man den Ester, das Reaktionsmedium und den Katalysator, der vorgebildet oder in Form seiner Komponenten eingesetzt wird, in den Reaktor und stellt durch Aufpressen von Kohlenmonoxid und Wasserstoff unter gleichzeitigem Aufheizen den gewünschten Druck ein. Es empfiehlt sich das Reaktionsgemisch zu rühren, sofern nicht bereits durch das Einleiten von Kohlenmonoxid und Wasserstoff ausreichende Durchmischung erzielt wird. Synthesegas wird dem Reaktor in dem Maße zugeführt, in dem es durch die Reaktion verbraucht wird.

Die Reaktion ist beendet, sobald keine Gasaufnahme mehr erfolgt.

Bevorzugt führt man die Umsetzung kontinuierlich durch. In diesem Fall werden Synthesegas, Crotonsäuremethylester und Reaktionsmedium fortlaufend in den das Katalysatorsystem enthaltenden Reaktor eingeleitet. Das Umsetzungsprodukt und ein Teil des Reaktionsmediums werden ständig abgezogen. Durch entsprechende Einstellung der Zufuhrgeschwindigkeit der Reaktionspartner und der Abnahmegeschwindigkeit der Produkte hält man in dem Reaktor einen stationären Zustand aufrecht. Eine Unterbrechung der Reaktion ist erst dann erforderlich, wenn die Katalysatorstabilität deutlich nachgelassen hat. Dieser Zeitpunkt kann dadurch hinausgezögert werden, daß man von Zeit zu Zeit einen Teil des gebrauchten Katalysators durch frischen ersetzt.

Die Reinigung des nach dem beanspruchten Verfahren hergestellten 2-Formylbuttersäuremethylesters erfolgt in bekannter Weise durch Destillation. Die Ausbeuten betragen über 85 %. Neben 2-Formylbuttersauremethylester enthält das Rohprodukt der Hydroformylierung etwa 2 bis 5 % 3-Formylbuttersäuremethylester und 5 bis 10 % Buttersäuremethylester.

In den folgenden Beispielen wird das neue Verfahren näher beschrieben.

### Beispiel 1

In einem 2 1-Autoklaven mit Hubrührer werden 500 g Crotonsäuremethylester, 500 g Cyclohexan, 0,6 g Triphenylphosphin und 100 ppm Rh (als Rh-2-ethylhexanoat) vorgelegt. Man läßt etwa 8 Stunden bei 90°C und einem CO/H₂-Druck von 27 MPa reagieren, kühlt darauf den Autoklaveninhalt ab und untersucht das Reaktionsgemisch durch Gaschromatographie.

Es ist wie folgt zusammengesetzt (in Gew.-%, ohne Berücksichtigung von Cyclohexan):

| | |
|---|---|
| Buttersäuremethylester | 4,23 |
| Crotonsäuremethylester | 0,59 |
| 2-Formylbuttersäuremethylester | 91,67 |
| 3-Formylbuttersäuremethylester | 2,05 |
| Sonstige | 1,46 |

### Beispiel 2

In einem 1 1-Autoklaven mit Hubrührer werden 250 g Crotonsäuremethylester, 250 g Cyclohexan, 0,83 g Triphenylphosphin und 100 ppm Rh (als Rh-2-ethylhexanoat) vorgelegt. Man läßt etwa 7 Stunden bei 90°C und einem CO/H₂-Druck von 27 MPa reagieren, kühlt darauf den Autoklaveninhalt ab und untersucht das Reaktionsgemisch durch Gaschromatographie.

Es ist wie folgt zusammengesetzt (in Gew.-%, ohne Berücksichtigung von Cyclohexan):

| | |
|---|---|
| Buttersäuremethylester | 5,23 |
| Crotonsäuremethylester | 0,11 |
| 2-Formylbuttersäuremethylester | 88,82 |
| 3-Formylbuttersäuremethylester | 4,21 |
| Sonstige | 1,63 |

### Beispiel 3

In einem 1 1-Autoklaven mit Hubrührer werden 250 g Crotonsäuremethylester, 250 g Cyclohexan, 0,83 g Triphenylphosphin und 100 ppm Rh (als Rh-2-ethylhexanoat) vorgelegt. Man läßt etwa 7 Stunden bei 100°C und einem CO/H₂-Druck von 28 MPa reagieren, kühlt darauf den Autoklaveninhalt ab und untersucht das Reaktionsgemisch durch Gaschromatographie.

Es ist wie folgt zusammengesetzt (in Gew.-%, ohne Berücksichtigung von Cyclohexan):

| | |
|---|---|
| Buttersäuremethylester | 7,11 |
| Crotonsäuremethylester | 0,08 |
| 2-Formylbuttersäuremethylester | 87,55 |
| 3-Formylbuttersäuremethylester | 4,01 |
| Sonstige | 1,25 |

## Patentansprüche

1. Verfahren zur Herstellung von 2-Formylbuttersäuremethylester durch Hydroformylierung von Crotonsäuremethylester in Gegenwart Rhodium und organische Phosphine enthaltender Katalysatoren, dadurch gekennzeichnet, daß die Umsetzung bei Temperaturen von 80 bis 120°C, Drücken von 20 bis 30 MPa, einer Rhodium-Konzentration von 10 bis 500 ppm, bezogen auf eingesetzten Crotonsäuremethylester, Tri-n-butylphosphin oder Triphenylphosphin als organischem Phosphin und in einem organischen Lösungsmittel als Reaktionsmedium erfolgt.

2. Verfahren nach Anspruch 1, dadurch gekennzeichent, daß die Konzentration des Rhodiums, bezogen auf den ursprünglich eingesetzten Crotonsäuremethylester, 50 bis 200 ppm beträgt.

3. Verfahren nach Anspruch 2, dadurch gekennzeichnet, daß die Konzentration des Rhodiums, bezogen auf den ursprünglich eingesetzten Crotonsäuremethylester, 80 bis 120 ppm beträgt.

4. Verfahren nach einem oder mehreren der Ansprüche 1 bis 3, dadurch gekennzeichnet, daß man je g-At Rhodium 1 bis 50 mol Phosphin anwendet.

5. Verfahren nach Anspruch 4, dadurch gekennzeichnet, daß man je g-At Rhodium 2 bis 20 mol Phosphin anwendet.

6. Verfahren nach einem oder mehreren der Ansprüche 1 bis 5, dadurch gekennzeichnet, daß die Hydroformylierung bei Temperaturen von 90 bis 110°C durchgeführt wird.

7. Verfahren nach Anspruch 6, dadurch gekennzeichnet, daß die Hydroformylierung bei Temperaturen von 90 bis 100°C durchgeführt wird.

8. Verfahren nach einem oder mehreren der Ansprüche 1 bis 7, dadurch gekennzeichnet, daß das organische Lösungsmittel ein aliphatischer Kohlenwasserstoff ist.

9. Verfahren nach Anspruch 8, dadurch gekennzeichnet, daß das organische Lösungsmittel Cyclohexan ist.

10. Verfahren nach einem oder mehreren der Ansprüche 1 bis 9, dadurch gekennzeichnet, daß der Anteil des organischen Lösungsmittels am Reaktionsgemisch 20 bis 80 Gew.-% beträgt.

## Claims

1. A process for preparing methyl 2-formyl-butyrate by hydroformylation of methyl crotonate in the presence of catalysts containing rhodium and organic phosphines, characterised in that the reaction takes place at temperatures of 80 to 120°C, at pressures of 20 to 30 MPa, with a rhodium concentration of 10 to 500 ppm, related to the methyl crotonate used, with tri-n-butylphosphine or triphenylphosphine as the organic phosphine and in an organic solvent as the reaction medium.

2. A process according to claim 1, characterised in that the concentration of the rhodium, related to the methyl crotonate originally used, is 50 to 200 ppm.

3. A process according to claim 2, characterised in that the concentration of the rhodium, related to the methyl crotonate originally used, is 80 to 120 ppm.

4. A process according to one or more of claims 1 to 3, characterised in that 1 to 50 mol of phosphine is used per gram-atom of rhodium.

5. A process according to claim 4, characterised in that 2 to 20 mol of phosphine is used per gram-atom of rhodium.

6. A process according to one or more of claims 1 to 5, characterised in that hydroformylation is carried out at temperatures of 90 to 110°C.

7. A process according to claim 6, characterised in that hydroformylation is carried out at temperatures of 90 to 100°C.

8. A process according to one or more of claims 1 to 7, characterised in that the organic solvent is an aliphatic hydrocarbon.

9. A process according to claim 8, characterised in that the organic solvent is cyclohexane.

10. A process according to one or more of claims 1 to 9, characterised in that the proportion of the organic solvent in the reaction mixture is 20 to 80 % by weight.

## Revendications

1. Procédé de préparation du 2-formylbutyrate de méthyle par hydroformylation du crotonate de méthyle en présence de catalyseurs contenant du rhodium et des phosphines organiques, caractérisé en ce que l'on réalise la réaction à des températures de 80 à 120°C, sous des pression de 30 à 30 MPa, à une concentration en rhodium de 10 à 500 ppm par rapport au crotonate de méthyle mis en oeuvre, avec de la tri-n-butylphosphine ou de la triphénylphosphine en tant que phosphine organique et dans un solvant organique qui sert de milieu de réaction.

2. Procédé selon la revendication 1, caractérisé en ce que la concentration du rhodium, par rapport au crotonate de méthyle mis en oeuvre à l'origine, est de 50 à 200 ppm.

3. Procédé selon la revendication 2, caractérisé en ce que la concentration du rhodium, par rapport au crotonate de méthyle mis en oeuvre à l'origine, est de 80 à 120 ppm.

4. Procédé selon une ou plusieurs des revendications 1 à 3, caractérisé en ce que l'on utilise de 1 à 50 mol de phosphine par atome-gramme de rhodium.

5. Procédé selon la revendication 4, caractérisé en ce que l'on utilise de 2 à 20 mol de phosphine par atome-gramme de rhodium.

6. Procédé selon une ou plusieurs des revendications 1 à 5, caractérisé en ce que l'on procéde à l'hydroformylation à des températures de 90 à 110°C.

7. Procédé selon la revendication 6, caractérisé en ce que l'on procède à l'hydroformylation à des températures de 90 à 100°C.

8. Procédé selon une ou plusieurs des revendications 1 à 7, caractérisé en ce que le solvant organique est un hydrocarbure aliphatique.

9. Procédé selon la revendication 8, caractérisé en ce que le solvant organique est le cyclohexane.

10. Procédé selon une ou plusieurs des revendications 1 à 9, caractérisé en ce que la proportion du solvant organique dans le mélange de réaction est de 20 à 80 % en poids.
